# EUROPEAN PATENT APPLICATION

(11) **EP 4 613 854 A1**
(43) Date of publication of application: **10.09.2025**
(21) Application number: 25159262.2
(22) Date of filing: 21.02.2025
(51) Int. Cl.: C12N 9/02, C07K 14/34, C12P 13/08, C12R 1/15

(54) **METHOD FOR THE FERMENTATIVE PRODUCTION OF L-LYSINE USING C. GLUTAMICUM STRAINS EXPRESSING A HETEROLOGOUS NICOTINAMIDE NUCLEOTIDE TRANSHYDROGENASE PNTAB AND HAVING AN INCREASED ACTIVITY OF A MYO-INOSITOL PERMEASE**

(30) Priority: 05.03.2024 EP 24161458
(71) Applicant: Evonik Operations GmbH, 45128 Essen (DE)
(72) Inventor: Kottenhahn, Patrick, 33602 Bielefeld (DE); Jerrentrup, Silke, 33607 Bielefeld (DE); Ramos Vera, Hugo, 33611 Bielefeld (DE)
(74) Representative: Evonik Patent Association

(57) **Abstract**

The present invention relates to a *C*. *glutamicum* strain which has enhanced productivity of L-lysine, and to a method for producing L-lysine using the same. In particular, the present invention concerns a *C*. *glutamicum* strain expressing NAD(P)(+) transhydrogenase subunit alpha PntA and NAD(P)(+) transhydrogenase subunit beta PntB combined with increased activity of the myo-inositol transporter IolT and a method for the fermentative production of L-lysine using such C. *glutamicum* strain.

## Description

The present invention relates to a *Corynebacterium glutamicum* (*C. glutamicum*) strain which has enhanced productivity of L-lysine, and to a method for producing L-lysine using the same. In particular, the present invention concerns a *C. glutamicum* strain expressing NAD(P)(+) transhydrogenase subunit alpha PntA and NAD(P)(+) transhydrogenase subunit beta PntB combined with increased activity of the myo-inositol transporter IoIT and a method for the fermentative production of L-lysine using such *C. glutamicum* strain.

Since L-Lysine remains a competitive market and has high commercial value, every percentage point increase in both production and productivity can bring huge benefits.

An important factor in the biotechnological production of L-lysine with microorganisms is the sufficient supply of NADPH. To produce one molecule of L-lysine, four molecules of NADPH are required to drive the necessary reducing steps. NADPH is provided from reactions of the pentose phosphate pathway (PPP) and the citric acid cycle (TCA).

To improve the availability of NADPH, production strains can be optimized by the expression of beneficial enzymes. For example, the introduction of an NADP-dependent glyceraldehyde 3-phosphate dehydrogenase (GapN) of *Streptococcus mutans* leads to increased NADPH availability in *Corynebacterium glutamicum* (*C. glutamicum*) and thus increased lysine yields (Takeno, S., Murata, R., Kobayashi, R., Mitsuhashi, S., & Ikeda, M. (2010) Applied and environmental microbiology, 76(21), 7154-7160). If an NADP dependent Gap enzyme is used instead of an NAD dependent one, energy conservation in form of ATP generation is omitted in this step, reducing the overall ATP yield per glucose.

Transhydrogenase activity offers an alternative perspective for sufficient NADPH supply, which is relevant for most amino acid production systems. One example is the use of the membrane-integral nicotinamide nucleotide transhydrogenase PntAB of *Escherichia coli* (*E.coli*), but also the use of PntAB of *Corynebacterium urealyticum* (*C. urealyticum*), which can use the electrochemical proton gradient across the cytoplasmic membrane to drive the reduction of NADP(+) via the oxidation of NADH. As *C. glutamicum* does not possess such an enzyme, the expression of the heterologous transhydrogenase PntAB gene results in an efficient way to improve the biotechnological production of metabolic compounds (Liu J, Li H, Zhao G, Caiyin Q, Qiao, J Ind Microbiol Biotechnol. 2018;45(5):313-327). Since the native reactions providing NADPH (*zwf, gnd, icd*) are associated with carbon loss in the form of CO₂ evolution, and GapN has a higher strain on energy conservation, use of PntAB is a preferable route in terms of ATP, carbon yield and environmental impact.

There are PntAB variants from different organisms available. The PntAB variant from *E. coli* is well researched and characterized. Further examples of organisms harbouring *pntAB* variants are *Oenococcus oeni*, *Synechocystis sp.* and *Lactobacillus buchneri* (Liu S, Skory C, Liang X, Mills D, Qureshi N., J Ind Microbiol Biotechnol. 2019;46(11):1547-1556). The *pntAB* variant from *Corynebacterium urealyticum* (*C. urealyticum*), a closer relative to *C. glutamicum*, has to date not been investigated in such detail.

It can be shown that the heterologous expression of *pntAB* for example from *E. coli* or from *C. urealyticum* in *C. glutamicum* leads to increased lysine yields (cf. Experimental Section). However, without adjustment of sugar translocation into the cell, the specific productivity of the cells does not increase, which in turn leads to longer fermentation runs. The term productivity is a measure of how fast a product (i.e. L-lysine in the present case) is being produced and is calculated as the concentration of product produced over time in grams product per liter per hour (g/L/h).

However, the reduction of the required fermentation times will lead to reduced production costs and higher space time yields, offering significant economic benefits.

The object of the present invention is to increase the productivity with respect to the production of L-lysine of a *C. glutamicum* strain comprising a heterologous gene encoding an enzyme with the function of a nicotinamide nucleotide transhydrogenase (PntAB), since PntAB on its own only leads to an increase of the L-lysine yield but has no positive effect on the productivity of said strain.

Therefore, the present invention concerns a *C. glutamicum* strain having the ability to produce L-lysine, comprising a heterologous gene encoding a NAD(P)(+) transhydrogenase subunit alpha (PntA), a heterologous gene encoding a NAD(P)(+) transhydrogenase subunit beta (PntB) and a gene encoding a myo-inositol permease (IoIT) that is overexpressed by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

A heterologous gene means that the gene has been inserted into a host organism which does not naturally have this gene. Insertion of the heterologous gene in the host is performed by recombinant DNA technology. Microorganisms that have undergone recombinant DNA technology are called transgenic, genetically modified or recombinant. Thus, the microorganism, i.e. The *C. glutamicum* strain, according to the present invention is recombinant.

In the context of the present invention, overexpression may be generally achieved by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

More particularly, overexpression of a gene can be achieved by increasing the copy number of the gene and/or by an enhancement of regulatory factors, e.g. by functionally linking the gene with a strong promoter and/or by enhancing the ribosomal binding site and/or by codon usage optimization of the start codon or of up to the whole gene. The enhancement of such regulatory factors which positively influence gene expression can, for example, be achieved by modifying the promoter sequence upstream of the structural gene in order to increase the effectiveness of the promoter or by completely replacing said promoter with a more effective or a so-called strong promoter. Promoters are located upstream of the gene. A promoter is a DNA sequence consisting of about 40 to 50 base pairs and which constitutes the binding site for an RNA polymerase holoenzyme and the transcriptional start point, whereby the strength of expression of the controlled polynucleotide or gene can be influenced. Generally, it is possible to achieve an overexpression or an increase in the expression of genes in bacteria by selecting strong promoters, for example by replacing the original promoter with strong, native (originally assigned to other genes) promoters or by modifying certain regions of a given, native promoter (for example its so-called -10 and -35 regions) towards a consensus sequence, e.g. as taught by M. Patek et al. (Microbial Biotechnology 6 (2013), 103-117) for *C. glutamicum.* An example for a "strong" promoter is the superoxide dismutase (*sod*) promoter ("Psod"; Z. Wang et al., Eng. Life Sci. 2015, 15, 73-82). A "functional linkage" is understood to mean the sequential arrangement of a promoter with a gene, which leads to a transcription of the gene. Another method to increase the expression of a target gene is the optimization of the codon utilization or translocation of the gene onto a more active region of the hosts genome. A further possibility for the overexpression of a gene is the deletion of a repressor or the mutation of the repressor binding region of the promotor, leading to constitutive expression of the gene.

The *C. glutamicum* strain according to the present invention is able to produce L-lysine. Preferably, the *C. glutamicum* strain according to the present invention is able to excrete L-lysine. L-lysine excreting strains of the species *C. glutamicum* are widely known in the art and can be used for the purpose of the present invention. For example, Blombach et al. (Applied and Environmental Microbiology 75(2), 419-427, 2009) describe the strain DM1933. Strain DM1933 was obtained from ATCC13032, the *C. glutamicum* type strain, by several steps of strain development. Other L-lysine excreting *C. glutamicum* strains are e. g. described in WO 2008033001 A1 and EP 0841395 A1.

The strain DM2042 is derived from DM1933 (Blombach et al., Applied and Environmental Microbiology 75(2), 419-427, 2009) with an increased activity of known beneficial lysine production genes *asd, dapA, dapB, lysA, lysC* (feedback resistant), *ddh* and *pyc.* These genes are overexpressed by use of a stronger promotor and/or by introduction of additional copies or variants of the respective genes to allow for unlimited flux through the lysine biosynthesis pathway.

A pure culture of the DM2042 strain has been deposited with the Deutsche Sammlung für Mikroorganismen and Zellkulturen (DSMZ, Braunschweig, Germany) as DSM 34720 in accordance with the Budapest Treaty on 2 August 2023.

Therefore, in the *C. glutamicum* strain according to the present invention the ability to produce L-lysine is preferably achieved by overexpression of the genes coding for enzymes having the function of aspartate-semialdehyde dehydrogenase (*asd*), aspartate aminotransferase (*aspB*), dihydrodipicolinate synthase (*dapA*), dihydrodipicolinate reductase (*dapB*), diaminopimelate decarboxylase (*lysA*), aspartokinase (*lysC*) and diaminopimelate dehydrogenase (*ddh*), wherein the overexpression is achieved by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

L-lysine excreting *C. glutamicum* strains typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild type strains such as ATCC13032, ATCC14067 and ATCC13869. The EC number for aspartokinase is EC 2.7.2.4. Descriptions of polynucleotides of *C. glutamicum* encoding a feedback resistant aspartokinase polypeptide variant are given for example in US5688671, US6844176 and US6893848. A summarizing list can be found inter alia in US20090311758A1.

The *C. glutamicum* strain according to the present invention may further comprise at least one copy of a gene *lysC* coding for a feedback resistant aspartokinase polypeptide variant. Preferably, the feedback resistant aspartokinase polypeptide variant comprises the amino acid sequence according to SEQ ID NO: 27. The amino acid sequence according to SEQ ID NO: 27 differs from the amino acid sequence of the wild type aspartokinase polypeptide of *Corynebacterium glutamicum* ATCC 13032 (the commercially available *C. glutamicum* type strain) in that the amino acid threonine (Thr) at position 311 is replaced by the amino acid isoleucine (Ile).

The wildtype DNA sequence of the *lysC* gene derived from *Corynebacterium glutamicum* ATCC 13032 encoding for aspartokinase can be achieved under NCBI Reference Sequence: NC_003450.3, locus_tag CGL_RS01330, also named NCgl0247. The according LysC polypeptide sequence derived from *Corynebacterium glutamicum* ATCC13032 is available under NCBI Reference Sequence: WP_003855724.1.

The *C. glutamicum* strain of the present invention may further comprise a gene coding for a pyruvate carboxylase (*pyc*) that is overexpressed by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

The nucleotide sequence of the *C. glutamicum* ATCC13032 chromosome and its analysis were described by Ikeda and Nakagawa (Applied Microbiology and Biotechnology 62, 99-109(2003)) and in EP1108790 A2. The information is available at the NCBI under accession number NC_003450.

The bacteria of the *C. glutamicum* strain of the present invention may comprise in their chromosome a heterologous polynucleotide encoding an amino acid sequence of a NAD(P)(+) transhydrogenase subunit alpha PntA comprising an amino acid sequence according to SEQ ID NO: 3 derived from *Escherichia coli* (*E. coli*) or SEQ ID NO: 7 derived from *Corynebacterium urealyticum* (*C. urealyticum*) and a heterologous polynucleotide encoding an amino acid sequence of a NAD(P)(+) transhydrogenase subunit beta PntB comprising an amino acid sequences according to SEQ ID NO: 4 derived from *E. coli* or SEQ ID NO: 8 derived from *C. urealyticum.*

The amino acid sequence comparison of PntA from *E. coli* SEQ ID NO: 3 and PntA from *C. urealyticum* SEQ ID NO: 7 results in an identity value of 57%.

The amino acid sequence comparison of PntB from *E. coli* SEQ ID NO: 4 and PntB from *C. urealyticum* SEQ ID NO: 8 results in an identity value of 66%.

In the *C. glutamicum* strain according to the present invention the heterologous gene encoding a NAD(P)(+) transhydrogenase subunit alpha is preferably coding for an amino acid sequence that has an identity of at least 60%, preferably of at least 90% to the amino acid sequences according to SEQ ID NO: 7. In a particular embodiment of the present invention the amino acid sequence of the NAD(P)(+) transhydrogenase subunit alpha is identical to the amino acid sequence according to SEQ ID NO: 7.

In the *C. glutamicum* strain according to the present invention the heterologous gene encoding a NAD(P)(+) transhydrogenase subunit beta is preferably coding for an amino acid sequence that has an identity of at least 70%, preferably of at least 90% to the amino acid sequences according to SEQ ID NO: 8. In a particular embodiment of the present invention the amino acid sequence of the NAD(P)(+) transhydrogenase subunit beta is identical to the amino acid sequence according to SEQ ID NO: 8.

lolT is a native myo-inositol permease of *C. glutamicum* that is also able to transport glucose into the cell as a side activity but unlike a PTS system, glucose is not phosphorylated by PEP during the transport. *C. glutamicum* harbors two lolT variants, IoIT1 and IoIT2, which have both been shown to be beneficial. Since both variants are reported to facilitate the same functionality with an amino acid sequence identity of IoIT2 to lolT1 of 57%, lolT2 was chosen to illustrate this invention as a representative for both variants (Lindner, Steffen N., et al., Applied and environmental microbiology, 2011, 77, Nr. 11, S. 3571-3581; Ikeda, Masato, et al. Applied microbiology and biotechnology, 2011, 90, S. 1443-1451.; XU, Jian-Zhong, et al., Microbial Cell Factories, 2020, 19, S. 1-15.).

SEQ ID NO: 11 shows the nucleotide sequence corresponding to *ioIT2* gene derived from *C. glutamicum* encoding a permease of the major facilitator superfamily (2), NCBI Reference Sequence: NC_003450.3, locus_tag CGL_RS15210, also named NCgl2953.

SEQ ID NO: 12 shows the IoIT2 polypeptide sequence derived from *C. glutamicum*, NCBI Reference Sequence: WP_011015598.1. In the *C. glutamicum* strain according to the present invention the overexpressed gene encoding a myo-inositol permease may encode an amino acid sequence which has an identity of 57% to the amino acid sequence according to SEQ ID NO: 12.

SEQ ID NO: 28 shows the nucleotide sequence of the *iolT1* gene derived from *C. glutamicum* encoding for the permease of the major facilitator superfamily (1), NCBI Reference Sequence: NC_003450.3, locus_tag CGL_RS00945, also named NCgl0178.

SEQ ID NO: 29 shows the IoIT1 polypeptide sequence derived from *C. glutamicum*, GenBank: WP_011013450.1.

Sequence identity of a conserved polynucleotide or polypeptide is determined by standard alignment algorithms, and may be used with a default gap penalty established by the program used. Substantially homologous or identical sequences are generally capable of hybridizing to all or part of a sequence under moderate or high stringent conditions. It is obvious that hybridization also includes hybridization with polynucleotides containing common codons in polynucleotides or codons taking codon degeneracy into account.

Whether any two polynucleotide or polypeptide sequences have homology, similarity or identity can be determined, for example, by Pearson et al (1988) [Proc. Natl. Acad. Sci. USA 85]: It can be determined using a known computer algorithm such as the "FASTA" program using default parameters as in 2444. Or, as performed in the Needleman program in the EMBOSS package (EMBOSS: The European Molecular Biology Open Software Suite, Rice et al., 2000, Trends Genet. 16: 276-277) (version 5.0.0 or later), It can be determined using the Needleman-Wunsch algorithm (Needleman and Wunsch, 1970, J. Mol. Biol. 48: 443-453) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop , [ed.,] Academic Press, San Diego, 1994, and [CARILLO ETA/.] (1988) SIAM J Applied Math 48: 1073. For example, BLAST from the National Center for Biotechnology Information Database, or Homology, similarity, or identity can be determined using ClustalW.

Identity of polynucleotides or polypeptides is defined in, for example, Smith and Waterman, Adv. Appl. Math (1981) 2:482, see, e.g., Needleman et al. (1970), J Mol Biol. This can be determined by comparing sequence information using a GAP computer program such as 48:443. In summary, a GAP program can be defined as the total number of symbols in the shorter of the two sequences divided by the number of similarly aligned symbols (i.e., nucleotides or amino acids). The default parameters for the GAP program are (1) a binary comparison matrix (containing values 1 for identity and 0 for non-identity) and Schwartz and Dayhoff, eds., Atlas Of Protein Sequence And Structure, National Biomedical Research Foundation , pp. 353-358 (1979), Gribskov et al (1986) Nucl. Acids Res. 14: Weighted comparison matrix of 6745 (or EDNAFULL (EMBOSS version of NCBI NUC4.4) permutation matrix); (2) a penalty of 3.0 for each gap and an additional 0.10 penalty for each symbol in each gap (or a gap opening penalty of 10 and a gap extension penalty of 0.5); and (3) no penalty for end gaps. Accordingly, the term "identity" used in this application refers to the relationship between sequences.

It was found that the *C. glutamicum* strain according to the present invention was able to excrete L-lysine into a suitable medium under suitable fermentation conditions in an increased manner with respect to e.g. product yield (in g L-lysine/I medium or g L-lysine/g carbon source) as well with increased productivity (in concentration of product produced over time in grams product per liter per hour, g/L/h) as compared to the unmodified *C. glutamicum* strain.

Therefore, the present invention also concerns a method for the fermentative production of L-lysine comprising the steps of cultivating the *C. glutamicum* strain of the present invention and accumulating L-lysine in the medium to form an L-lysine containing fermentation broth.

The term L-lysine, where mentioned herein, in particular in the context of product formation, also comprises their ionic forms and salts, for example L-lysine mono hydrochloride or L-lysine sulfate.

The method according to the present invention may further comprise manufacturing an L-lysine containing product from said fermentation broth or isolating L-lysine from the L-lysine containing fermentation broth. In a fermentative process according to the invention the *C. glutamicum* strain modified in accordance with the present invention, and having the ability to excrete L-lysine is cultivated in a suitable medium under suitable conditions. Due to said ability to excrete said L-lysine the concentration of the L-lysine increases and accumulates in the medium during the fermentative process and the L-lysine is thus produced. Further aspects on fermentation process parameters according to the invention are described e.g. in EP3686277 A1.

### Short description of the sequences

SEQ ID NO: 1 DNA sequence of the *pntA* gene derived from *Escherichia coli* encoding the pyridine nucleotide transhydrogenase subunit alpha, *pntA:* NCBI-GeneID: 946628.
SEQ ID NO: 2 DNA sequence of the *pntB* gene derived from *Escherichia coli* encoding the pyridine nucleotide transhydrogenase subunit beta, *pntB:* NCBI-GeneID: 946144.
SEQ ID NO: 3 PntA polypeptide sequence derived from *Escherichia coli,* NCBI Reference Sequence: PntA: NP_416120.1.
SEQ ID NO: 4 PntB polypeptide sequence derived from *Escherichia coli,* NCBI Reference Sequence: PntB: NP_416119.1.
SEQ ID NO: 5 DNA sequence of the *pntA* gene derived from *Corynebacterium urealyticum* encoding the NAD(P)(+) transhydrogenase subunit alpha, NCBI Reference Sequence: *pntA:* CKV82_RS04765.
SEQ ID NO: 6 DNA sequence of the *pntB* gene derived from *Corynebacterium urealyticum* encoding the NAD(P)(+) transhydrogenase subunit beta, NCBI Reference Sequence: *pntB:* CKV82_RS04770.
SEQ ID NO: 7 PntA polypeptide sequence derived from *Corynebacterium urealyticum,* NCBI Reference Sequence: PntA: WP_012360758.1.
SEQ ID NO: 8 PntB polypeptide sequence derived from *Corynebacterium urealyticum,* NCBI Reference Sequence: PntB: WP_012360759.1.
SEQ ID NO: 9 *pntAB_ec* polynucleotide carrying intergenic sequences derived from *Corynebacterium glutamicum*, promotor P2 (base pair position 807-986), codon optimized variant of *pntA* and *pntB* genes derived from *Escherichia coli.*
SEQ ID NO: 10 *pntAB_cu* polynucleotide carrying intergenic sequences derived from *Corynebacterium glutamicum*, promotor P2 (base pair position 807-986), *pntA* and *pntB* genes derived from *Corynebacterium urealyticum.*
SEQ ID NO: 11 DNA sequence of the *ioIT2* gene derived from *Corynebacterium glutamicum* encoding for a permease of the major facilitator superfamily 2, NCBI Reference Sequence: NC_003450.3, locus_tag CGL_RS15210, also named NCgl2953
SEQ ID NO: 12 loIT2 polypeptide sequence derived from *Corynebacterium glutamicum*, NCBI Reference Sequence: WP_011015598.1.
SEQ ID NO: 13 P1-*iolT2* polynucleotide carrying intergenic sequences, promotor P1 (base pair position 792-911) and initial sequence of the *ioIT2* gene derived from *Corynebacterium* glutamicum.
SEQ ID NO: 14 DNA sequence of the *ptsG* gene derived from *Corynebacterium glutamicum* encoding for the glucose-specific phosphotransferase system enzyme II, GenBank: CGL_RS06785.
SEQ ID NO: 15 PtsG polypeptide sequence derived from *Corynebacterium glutamicum*, GenBank: ABB53258.1.
SEQ ID NO: 16 P2-ptsG polynucleotide carrying intergenic sequences, promotor P2 (base pair position 751-930) and initial sequence of the *ptsG* gene derived from *Corynebacterium glutamicum.*
SEQ ID NO: 17 DNA sequence of the *glf* gene derived from *Zymomonas mobilis* encoding a glucose transport protein, GenBank: M60615.1, base pair position 185-1606
SEQ ID NO: 18 Glf polypeptide sequence derived from *Zymomonas mobilis*, Proteine ID: AAA27691.1
SEQ ID NO: 19 P1-glf polynucleotide carrying intergenic sequences derived from *Corynebacterium glutamicum*, promotor P1 (base pair position 792-911) and *glf* gene derived from *Zymomonas mobilis.*
SEQ ID NO: 20 DNA sequence of the *galP* gene derived from *Escherichia coli* encoding a galactose:H(+) symporter, Gene ID: 947434.
SEQ ID NO: 21 GalP polypeptide sequence derived from *Escherichia coli*, NCBI Reference Sequence: NP_417418.1
SEQ ID NO: 22 *P1-galP* polynucleotide carrying intergenic sequences derived from *Corynebacterium glutamicum*, promotor P1 (base pair position 792-911) and *galP* gene derived from *Escherichia coli.*
SEQ ID NO: 23 DNA sequence of the *bglF* gene derived from *Corynebacterium glutamicum R* encoding Ell component of the β-glucoside-PTS, GenBank: AF508972.1, base pair position 1320-3176.
SEQ ID NO: 24 BglF polypeptide sequence derived from *Corynebacterium glutamicum R*, GenBank: AAO15901.1.
SEQ ID NO: 25 *P1-bglF* polynucleotide carrying intergenic sequences derived from *Corynebacterium glutamicum*, promotor P1 (base pair position 792-911) and *bglF* gene derived from *Corynebacterium glutamicum R.*
SEQ ID NO: 26 DNA sequence of the *lysC* gene derived from *Corynebacterium glutamicum* encoding the feedback resistant aspartokinase polypeptide variant LysC_T311I
SEQ ID NO: 27 LysC_T311I polypeptide sequence derived from *Corynebacterium glutamicum*,
SEQ ID NO: 28 DNA sequence of the *iolT1* gene derived from *Corynebacterium glutamicum* encoding for the permease of the major facilitator superfamily 1, NCBI Reference Sequence: NC_003450.3, locus_tag CGL_RS00945, also named NCgl0178
SEQ ID NO: 29 lolT1 polypeptide sequence derived from *Corynebacterium glutamicum*, GenBank: WP_011013450.1

The term of intergenic sequences used herein implies homologous sequences which are used to enable the replacement of native promotor regions or to incorporate heterologous genes by homologous recombination.

### EXPERIMENTAL SECTION

### A) MATERIALS and METHODS

The molecular biology kits, primers and chemicals used and some details of the methods applied are briefly described herewith.

### 1. Antibiotics and chemicals

a. Kanamycin: Kanamycin solution from *Streptomyces kanamyceticus* from Sigma Aldrich (St. Louis, USA, Cat. no. K0254).
b. Nalidixic acid: Nalidixic acid sodium salt from Sigma Aldrich (St. Louis, USA, Cat. no. N4382).
c. If not stated otherwise, all chemicals were purchased analytically pure from Merck (Darmstadt, Germany), Sigma Aldrich (St. Louis, USA) or Carl-Roth (Karlsruhe, Germany).

### 2. Cultivation

If not stated otherwise, all cultivation / incubation procedures were performed as follows herewith:
a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 37°C and 200 rpm.
b. LB agar (MILLER) from Merck (Darmstadt, Germany Cat. no. 110283) was used for cultivation of *E. coli* strains on agar plates. The agar plates were incubated at 37°C in an INCU-Line^{®} mini-incubator from VWR (Radnor, USA).
c. Brain heart infusion broth (BHI) from Merck (Darmstadt, Germany; Cat. no. 110493) was used to cultivate *C. glutamicum* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 33°C and 200 rpm.
d. Brain heart agar (BHI-agar) from Merck (Darmstadt, Germany; Cat. no. 113825) was used for cultivation of *C. glutamicum* strains on agar plates. The agar plates were incubated at 33°C in an incubator from Heraeus Instruments with Kelvitron^{®} temperature controller (Hanau, Germany).

### 3. Determining optical density

a. The optical density of bacterial suspensions in shake flask cultures was determined at 600 nm (OD600) using the BioPhotometer from Eppendorf AG (Hamburg, Germany).
b. The optical density of bacterial suspensions produced in the Wouter Duetz (WDS) micro fermentation system (24-Well Plates) was determined at 660 nm (OD660) with the GENios^{™} plate reader from Tecan Group AG (Männedorf, Switzerland).

### 4. Centrifugation

a. Benchtop centrifuge for reaction tubes with a volume up to 2 ml Bacterial suspensions with a maximum volume of 2 ml were caused to sediment using 1 ml or 2 ml reaction tubes (e.g., Eppendorf Tubes^{®} 3810X) using an Eppendorf 5417 R centrifuge (5 min at 13.000 rpm).
b. Benchtop centrifuge for tubes with a volume up to 50 ml Bacterial suspensions with a maximum volume of 50 ml were caused to sediment using 15 ml or 50 ml centrifuge tubes (e.g. Falcon^{™} 50 ml Conical Centrifuge Tubes) using an Eppendorf 5810 R centrifuge for 10 min at 4.000 rpm.

### 5. PCR

Integration of desired DNA fragments into the chromosome of *C. glutamicum* was detected by PCR amplification and following Sanger sequencing of the PCR product.
a. Primers
The oligonucleotides used were synthesized by eurofins genomics GmbH (Ebersberg, Germany).
b. Template
As PCR template the total DNA contained in a colony was used. It was prepared by taking cell material with a toothpick from a colony on an agar plate and placing the cell material directly into the PCR reaction tube. The cell material was heated for 10 sec with 800 W in a microwave oven type Mikrowave & Grill from SEVERIN Elektrogeräte GmbH (Sundern, Germany) and then the PCR reagents were added to the template in the PCR reaction tube.
b. Reaction Mix
The PCR reaction was carried out using SapphireAmp^{®} Fast PCR Master Mix from Takara Bio Inc. (Shiga, Japan). The reaction mixture was prepared according to the manufacturer's information. Therefore 25 µl of the SapphireAmp^{®} Fast PCR Master Mix (2x) was mixed with 0.5 µl of each of the PCR-Primers [100 pmol/µl] and 24 µl H₂O to get in total 50 µl reaction volume. The thermocycling conditions for PCR are shown in Table 1.

**Table 1: Thermocycling conditions for PCR**

| PCR-program | | | |
|---|---|---|---|
| Step | Time [sec] | T [°C] | Description |
| 1 | 60 | 94 | Denaturation |
| | | | Amplification |
| 2 | 05 | 98 | Denaturation step |
| 3 | 05 | 55 | Annealing step |
| 4 | 10 sec/kb | 72 | Elongation step |
| | | | Repeat step 2 to 4: 30 x |

c. PCR Cycler
The reactions were carried out in a Mastercycler^{®} of Eppendorf (Hamburg, Germany).

### 6. Chemical transformation of E. coli

*E. coli* K-12 strain S17-1 was used as donor for conjugational transfer of plasmids based on pK18mobsacB from E. *coli* to *C. glutamicum.* Strain S17-1 is described by Simon, R. et al. (Bio/Technology 1, 784-794, 1983). It is available from the American Type Culture Collection under the access number ATCC47055.

Chemically competent E. *coli* S17-1 cells were made as follows: A preculture of 10 ml LB medium (10 ml liquid medium per 100 ml Erlenmeyer flask with 3 baffles) was inoculated with 100 µl bacterial suspension of strain S17-1 and the culture was incubated overnight for about 18 h at 37°C and 250 rpm. The main culture (70 ml LB contained in a 250 ml Erlenmeyer flask with 3 baffles) was inoculated with 300 µl of the preculture and incubated up to an OD600 of 0.5-0.8 at 37°C. The culture was centrifuged for 6 min at 4°C and 4000 rpm and the supernatant was discarded. The cell pellet was resuspended in 20 ml sterile, ice-cold 50 mM CaCl₂ solution and incubated on ice for 30 min. After another centrifugation step, the pellet was resuspended in 5 ml ice-cold 50 mM CaCl₂ solution and the suspension incubated on ice for 30 min. The cell suspension was then adjusted to a final concentration of 20 % glycerol (v/v) with 85 % (v/v) sterile ice-cold glycerol. The suspension was divided into 50 µl aliquots and stored at -80°C.

To transform S17-1 cells, the protocol according to Tang et al. (Nucleic Acids Res. 22(14), 2857-2858, 1994) with a heat shock of 45 sec. was used.

### 7. Conjugation of C. glutamicum

The pK18mobsacB plasmid system described by Schäfer et al. (Gene 145, 69 - 73, 1994) was used to integrate desired DNA fragments into the chromosome of *C. glutamicum.* A modified conjugation method of Schäfer et al. (Journal of Bacteriology 172, 1663 - 1666, 1990) was used to transfer the respective plasmid into the desired *C. glutamicum* recipient strain.

Liquid cultures of the *C. glutamicum* strains were carried out in BHI medium at 33°C. The heat shock was carried out at 48.5°C for 9 min. Transconjugants were selected by plating the conjugation batch on EM8 agar (Table 2), which was supplemented with 25 mg/l kanamycin and 50 mg/l nalidixic acid. The EM8 agar plates were incubated for 72 h at 33°C.

**Table 2: Composition of the EM8 agar**

| Components | Concentration (g/l) |
|---|---|
| Glucose (sterile filtered) | 23 |
| CSL (corn steep liquor; Roquette; solid content 48±2 % w/w) | 30 |
| Peptone from soymeal (Merck, Germany) | 40 |
| (NH₄)₂SO₄ | 8 |
| Urea | 3 |
| KH₂PO₄ | 4 |
| MgSO₄ · 7 H₂O | 0.5 |
| FeSO₄ · 7 H₂O | 0.01 |
| CuSO₄ · 5 H₂O | 0.001 |
| ZnSO₄ · 7 H₂O | 0.01 |
| Calcium pantothenate, D(+) | 0.01 |
| Thiamine | 0.001 |
| Inositol | 0.1 |
| Nicotinic acid | 0.001 |
| Biotin (sterile filtered) | 0.005 |
| CaCO₃ (autoclaved separately) | 1.6 |
| Agar-Agar (Merck, Germany) | 14 |

Sterile toothpicks were used to transfer the transconjugants onto BHI agar, which was supplemented with 25 mg/l kanamycin and 50 mg/l nalidixic acid. The agar plates were incubated for 20 h at 33°C. The cultures of the respective transconjugants produced in this manner were then propagated further for 24 h at 33°C in 10 ml BHI medium contained in 100 ml Erlenmeyer flasks with 3 baffles. An aliquot was taken from the liquid culture suitably diluted and plated (typically 100 to 200 µl) on BHI agar which was supplemented with 10% saccharose. The agar plates were incubated for 48 h at 33°C. The colonies growing on the saccharose containing agar plates were then examined for the phenotype kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined for integration of the desired DNA fragment by means of PCR and following sequencing of the PCR product.

### 8. Glycerol stocks of E. coli and C. glutamicum strains

For long time storage of *E. coli-* and *C. glutamicum* strains glycerol stocks were prepared. Selected *E. coli* clones were cultivated in 10 ml LB medium supplemented with 2 g/l glucose. Selected *C. glutamicum* clones were cultivated in two-fold concentrated BHI medium supplemented with 2 g/l glucose. Cultures of plasmid containing *E. coli* strains were supplemented with 50 mg/l kanamycin. Cultures of plasmid containing *C. glutamicum* strains were supplemented with 25 mg/l kanamycin. The medium was contained in 100 ml Erlenmeyer flasks with 3 baffles. It was inoculated with a loop of cells taken from a colony and the culture incubated for about 18 h at 37°C and 200 rpm in the case of E. *coli* and 33°C and 200 rpm in the case of *C. glutamicum.* After said incubation period 1.2 ml 85% (v/v) sterile glycerol were added to the culture. The obtained glycerol containing cell suspension was then aliquoted in 2 ml portions and stored at -80°C.

### 9. Cultivation system according to Wouter Duetz (WDS)

The millilitre-scale cultivation system according to Duetz (Trends Microbiol. 2007; 15(10):469-75) was used to investigate the performance of the *C. glutamicum* strains constructed. For this purpose, 24-deepwell microplates (24 well WDS plates) from EnzyScreen BV (Heemstede, Netherlands; Cat. no. CR1424), filled with 2.5 ml medium were used.

Precultures of the strains were done in 10 ml two-fold concentrated BHI medium. The medium was contained in a 100 ml Erlenmeyer flask with 3 baffles. It was inoculated with 100 µl of a glycerol stock culture and the culture incubated for 24 h at 33°C and 200 rpm.

After said incubation period the optical densities OD600 of the precultures were determined.

The main cultures were done by inoculating the 2.5 ml medium containing wells of the 24 Well WDS-Plate with an aliquot of the preculture to give an optical density OD600 of 0.1.

As medium for the main culture CGXII medium (described by Keilhauer et al. (J. Bacteriol. 1993 Sep; 175(17): 5595-5603)) with 7,5 g/l CSL and 20 g/l glucose was used. The composition of the modified CGXII medium is shown in Table 3.

**Table 3: Composition of Keilhauer's CGXII medium (modified)**

| Components | Concentration (g/l) |
|---|---|
| MOPS (3-(N-Morpholino)propanesulfonic acid) | 42 |
| (NH₄)₂SO₄ | 20 |
| Urea | 5 |
| KH₂PO₄ | 1 |
| K₂HPO₄ | 1 |
| MgSO₄ · 7 H₂O | 0.25 |
| CaCl₂ | 0.01 |
| FeSO₄ · 7 H₂O | 0.01 |
| MnSO₄ H₂O | 0.01 |
| ZnSO₄ · 7 H₂O | 0.001 |
| CuSO₄ · 5 H₂O | 0.0002 |
| NiCl₂ 6 H₂O | 0.00002 |
| Biotin (sterile-filtered) | 0.0002 |
| Protocatechuic acid (sterile-filtered) | 0.03 |
| CSL (corn steep liquor; Roquette; solid content 50 % w/w) | 7.5 |
| Glucose (sterile filtered) | 20.0 |
| adjust the pH to 7 with NaOH | |

These main cultures were incubated for approximately 45 h at 33 °C and 300 rpm in an Infors HT Multitron standard incubator shaker from Infors GmbH (Bottmingen, Switzerland) until complete consumption of glucose.

The glucose concentration in the suspension was analyzed with the blood glucose-meter OneTouch Vita^{®} from LifeScan (Johnson & Johnson Medical GmbH, Neuss, Germany).

After cultivation the culture suspensions were transferred to a deep well microplate. A part of the culture suspension was suitably diluted to measure the OD660. Another part of the culture was centrifuged and the concentration of L-amino acids, in particular L-lysine, and residual glucose were analyzed in the supernatant.

### 10. Amino acid analyser

The concentration of L-lysine and other L-amino acids, e.g. L-valine, in the culture supernatants was determined by ion exchange chromatography using a SYKAM S433 amino acid analyser from SYKAM Vertriebs GmbH (Fürstenfeldbruck, Germany). As solid phase a column with spherical, polystyrene-based cation exchanger (Peek LCA N04/Na, dimension 150 × 4.6 mm) from SYKAM was used. Depending on the L-amino acid the separation takes place in an isocratic run using a mixture of buffers A and B for elution or by gradient elution using said buffers. As buffer A an aqueous solution containing in 20 l 263 g trisodium citrate, 120 g citric acid, 1100 ml methanol, 100 ml 37 % HCl and 2 ml octanoic acid (final pH 3.5) was used. As buffer B an aqueous solution containing in 20 l 392 g trisodium citrate, 100 g boric acid and 2 ml octanoic acid (final pH 10.2) was used. The free amino acids were coloured with ninhydrin through post-column derivatization and detected photometrically at 570 nm.

### 11. Glucose determination with continuous flow system (CFS)

A SANplus multi-channel continuous flow analyser from SKALAR analytic GmbH (Erkelenz, Germany) was used to determine the concentration of glucose in the supernatant. Glucose was detected with a coupled-enzyme assay (Hexokinase/ Glucose-6-Phosphate-Dehydrogenase) via NADH formation.

### B) EXPERIMENTAL RESULTS

### Example 1

Sequence of the *pntA and pntB* genes derived from *Escherichia coli* and *pntA and pntB* genes derived from *Corynebacterium urealyticum*

The *pntA* (SEQ ID NO: 1) and *pntB* (SEQ ID NO: 2) genes derived from *Escherichia coli* code for a pyridine nucleotide transhydrogenase subunit alpha and pyridine nucleotide transhydrogenase subunit beta, respectively. The encoded polypeptide sequence can be found at the NCBI under NCBI Reference Sequence: *pntA:* NP_416120.1 (SEQ ID NO: 3), *pntB:* NP_416119.1 (SEQ ID NO: 4).

The *pntA* (NCBI Reference Sequence: CKV82_RS04765) (SEQ ID NO: 5) *and pntB* (NCBI Reference Sequence: CKV82_RS04770) (SEQ ID NO: 6) genes derived from *Corynebacterium urealyticum* code for a NAD(P)(+) transhydrogenase subunit alpha and NAD(P)(+) transhydrogenase subunit beta, respectively. The encoded polypeptide sequence can be found at the NCBI under the description Re/Si-specific NAD(P)(+) transhydrogenase subunit alpha as NCBI Reference Sequence: *pntA*: WP_012360758.1 (SEQ ID NO: 7) and *pntB:* WP_012360759.1 (SEQ ID NO: 8) under the description NAD(P)(+) transhydrogenase (Re/Si-specific) subunit beta.

### Example 2

### Construction of plasmids pK18mobsacB_pntAB_ec and pK18mobsacB_pntAB_cu

Plasmids pK18mobsacB_*pntAB*_ec and pK18mobsacB_*pntAB*_cu were constructed to enable incorporation of the heterologous *pntAB* genes *pntAB_ec* and *pntAB_cu* in lysine producing strain DM2042. The plasmids are based on the mobilizable vector pK18mobsacB described by Schäfer et al. (Gene 145, 69-73, 1994). For construction of pK18mobsacB_*pntAB*_ec and pK18mobsacB_pntAB_cu, the *pntAB_ec* polynucleotide according to SEQ ID NO: 9 (carrying intergenic sequences, promotor P2, *pntA* gene and *pntB* gene derived from *Escherichia coli*) and the *pntAB_cu* polynucleotide according to SEQ ID NO: 10 (carrying intergenic sequences, promotor P2, *pntA* gene and *pntB* gene derived from *Corynebacterium urealyticum*) were synthesized and subcloned into pK18mobsacB by GeneArt (ThermoFisher Scientific (Waltham, USA)).

The intergenic sequences of SEQ ID NO: 9 and SEQ ID NO: 10 derived from *Corynebacterium glutamicum* represent homologous sequences which enable to incorporate the heterologous *pntA* and *pntB* genes by homologous recombination.

Seq ID NO: 9 represents the codon optimized variant of *pntA* and *pntB* gene sequences which were used for construction the plasmid pK18mobsacB_*pntAB*_ec.

Seq ID NO: 10 comprises an ATG nucleotide as start codon which is a variant of the native *pntA* gene sequence derived from *Corynebacterium urealyticum* which was used for construction the plasmid pK18mobsacB_*pntAB*_cu.

To assemble the plasmids pK18mobsacB_*pntAB*_ec the following steps were done by GeneArt: The two polynucleotides i.e., the vector pK18mobsacB and the polynucleotide *pntAB_ec* were treated with Xmal and Sbfl, ligated and the ligation mixture used to transform *E. coli.*

To assemble the plasmids pK18mobsacB_pntAB_cu the following steps were done by GeneArt: The two polynucleotides i.e., the vector pK18mobsacB and the polynucleotide *pntAB_cu* were treated with Xbal and Sbfl, ligated and the ligation mixture used to transform *E. coli.*

DNA of plasmids pK18mobsacB_*pntAB*_ec and pK18mobsacB_*pntAB*_cu was isolated from a transformant and the polynucleotides *pntAB_ec* and *pntAB*_cu, respectively, created within pK18mobsacB was analyzed by Sanger sequencing.

### Example 3

### Construction of strains DM2042_pntAB_ec and DM2042_pntAB_cu

Strain DM1933 is an L-lysine producer derived from the *C. glutamicum* wild type strain ATCC 13032 and has been described by Blombach et al. (Applied and Environmental Microbiology 75(2), 419-427, 2009).

Strain DM2042 is derived from DM1933, with an increased activity of known beneficial lysine production genes *asd, dapA, dapB, lysA, lysC* (feedback resistant), *ddh* and *pyc.* These genes are overexpressed by use of a stronger promotor and/or introduction of additional copies or variants of the respective gene to allow for unlimited flux through the lysine biosynthesis pathway.

A pure culture of the DM2042 strain has been deposited with the Deutsche Sammlung für Mikroorganismen and Zellkulturen (DSMZ, Braunschweig, Germany) as DSM 34720 in accordance with the Budapest Treaty on 2 August 2023.

The plasmids pK18mobsacB_*pntAB*_ec and pK18mobsacB_*pntAB*_cu obtained in example 2 were used to incorporate the heterologous genes *pntAB_ec* derived from *E. coli* and *pntAB_cu* derived from *C. urealyticum,* respectively.

Chemically competent cells of *E. coli* strain S17-1 were transformed with plasmid DNA of pK18mobsacB_*pntAB*_ec and pK18mobsacB_*pntAB*_cu. The presence of the plasmids obtained in example 2 were analyzed by Sanger sequencing, respectively.

For conjugal transfer into the strain DM2042 the modified conjugation method of Schäfer et al. (Journal of Bacteriology 172, 1663 - 1666, 1990) as described in materials and methods was used. Subsequently selection of transconjugant clones was done by identification of saccharose resistance and kanamycin sensitivity phenotype.

Transconjugant clones of DM2042 carrying the heterologous *pntAB* genes *pntAB_ec* or *pntAB*_cu were selected according to chromosome sequence analysis. The strain carrying the heterologous *pntAB*_ec gene was called DM2042_*pntAB*_ec. The strain carrying the heterologous *pntAB*_cu gene was called DM2042_*pntAB*_cu.

A glycerol stock culture of the transconjugant clone was prepared and used as starting material for further investigations.

### Example 4

Sequence of *ioIT2* and *ptsG* genes derived from *Corynebacterium glutamicum.*

The nucleotide sequence of *ioIT2* (CGL_RS15210) and *ptsG* (CGL_RS06785) genes derived from *Corynebacterium glutamicum* as well as the encoded polypeptide sequences can be found at the NCBI. The corresponding NCBI Reference Sequences are listed below in Table 4.

**Table 4: ioIT2 and ptsG genes derived from Corynebacterium glutamicum**

| gene | | | polypeptide sequences | | | | organism |
|---|---|---|---|---|---|---|---|
| name | SEQ ID | NCBI / GenBank ID | name | SEQ ID | NCBI reference sequence / GenBank ID | function | |
| *iolT2* | 11 | NC_003450.3, locus_tag CGL_RS15210 | IoIT2 | 12 | WP_011015598.1 | permease of the major facilitator superfamily | *C. glutamicum* |
| *ptsG* | 14 | NC_003450.3, locus_tag CGL_RS06785 | PtsG | 15 | WP_011014304.1 | glucose-specific phosphotransf erase system enzyme II | *C. glutamicum* |

### Example 5

### Construction of plasmids pK18mobsacB_P1-ioIT and pK18mobsacB_P2-ptsG

Plasmids pK18mobsacB_P1-*ioIT* and pK18mobsacB_P2-ptsG were constructed to enable replacement of the nucleotide sequence upstream to *ioIT2* and *ptsG* respectively with the sequence of promotor P1 and P2 in lysine producing strains DM2042, DM2042_*pntAB*_ec and DM2042_*pntAB*_cu. The plasmids are based on the mobilizable vector pK18mobsacB described by Schäfer et al. (Gene 145, 69-73, 1994). For construction of pK18mobsacB_P1-*ioIT* and pK18mobsacB_P2-*ptsG*, the P1-*ioIT* polynucleotide according to SEQ ID NO: 13 (carrying intergenic sequences, promotor P1 and *ioIT2* gene derived from *Corynebacterium glutamicum*) and the P2-ptsG polynucleotide according to SEQ ID NO: 16 (carrying intergenic sequences, promotor P2 and *ptsG* gene derived from *Corynebacterium glutamicum*) were synthesized and subcloned into pK18mobsacB by GeneArt (ThermoFisher Scientific (Waltham, USA)).

The intergenic sequences of SEQ ID NO: 13 and SEQ ID NO: 16 derived from *Corynebacterium glutamicum* represent homologous sequences which enable to replace the native promotor region of *ioIT2* and *ptsG* respectively by homologous recombination.

To assemble the plasmids pK18mobsacB_P1-*ioIT* and pK18mobsacB_P2-*ptsG* the following steps were done by GeneArt: The two polynucleotides i.e., the vector pK18mobsacB and the corresponding polynucleotide P1-*ioIT* and P2-*ptsG* were treated with 2 restriction enzymes (see Table 5 below), ligated and the ligation mixture used to transform *E. coli.*

DNA of plasmids pK18mobsacB_P1-*ioIT* and pK18mobsacB_P2-*ptsG* were isolated from transformants and the polynucleotide P1-*ioIT* and P2-*ptsG* created within pK18mobsacB were analyzed by Sanger sequencing.

**Table 5: Construction of plasmids pK18mobsacB_P1-ioIT and pK18mobsacB_P2 -ptsG**

| polynucleotide | | construction | |
|---|---|---|---|
| name | SEQ ID | restriction enzymes | construct |
| P1- *ioIT* | 13 | BamHI, Sbfl | pK18mobsacB_P1-*iolT* |
| P2- *ptsG* | 16 | Xbal, Sbfl | pK18mobsacB_P2-*ptsG* |

### Example 6

### Construction of strains DM2042_P1-ioIT, DM2042_pntAB_ec_P1-ioIT, DM2042_pntAB_cu_P1-ioIT, DM2042_P2-ptsG and DM2042_pntAB_cu_P2-ptsG

The plasmids pK18mobsacB_P1-*ioIT* and pK18mobsacB_P2-*ptsG* obtained in example 5 were used to replace the native promotor regions upstream of the *ioIT2* gene derived from *C. glutamicum* and the *ptsG* gene derived from *C. glutamicum,* respectively.

Chemically competent cells of *E. coli* strain S17-1 were transformed with plasmid DNA of pK18mobsacB_P1-*ioIT* and pK18mobsacB_P2-*ptsG*. The presence of the plasmids obtained in example 5 were analyzed by Sanger sequencing, respectively.

For conjugal transfer into the strains DM2042, DM2042_*pntAB*_ec and DM2042_*pntAB*_cu the modified conjugation method of Schäfer et al. (Journal of Bacteriology 172, 1663 - 1666, 1990) as described in materials and methods was used. Subsequently, selection of transconjugant clones was done by identification of saccharose resistance and kanamycin sensitivity phenotype. Transconjugant clones of DM2042, DM2042_*pntAB*_ec and DM2042_*pntAB*_cu carrying the replaced P1 Promotor upstream to *ioIT2* were selected according to chromosome sequence analysis. The respective strains carrying the replaced P1 Promotor upstream to *ioIT2* were called DM2042_P1-*ioIT*, DM2042_*pntAB*_ec_P1-*ioIT* and DM2042_*pntAB*_cu_P1-*ioIT.*

Transconjugant clones of DM2042 and DM2042_*pntAB*_cu carrying the replaced P2 Promotor upstream to *ptsG* were selected according to chromosome sequence analysis. The respective strains carrying the replaced P2 Promotor upstream to *ptsG* were called DM2042_P2-*ptsG* and DM2042_*pntAB*_cu_P2-*ptsG*.

Glycerol stock cultures of the transconjugant clones were prepared and used as starting material for further investigations.

### Example 7

Sequence of *glf* gene derived from *Zymomonas mobilis, galP* gene derived from *Escherichia coli and bglF* gene derived from *Corynebacterium glutamicum R.*

The nucleotide sequence of the *glf* gene derived from *Zymomonas mobilis,* the *galP* gene derived from *Escherichia coli and bglF* gene derived from *Corynebacterium glutamicum R* as well as the encoded polypeptide sequences can be found at the NCBI. The corresponding NCBI Reference Sequences are listed below in Table 6.

**Table 6: glf gene derived from Zymomonas mobilis, galP gene derived from Escherichia coli and bglF gene derived from Corynebacterium glutamicum R.**

| gene | | | polypeptide sequences | | | | organism |
|---|---|---|---|---|---|---|---|
| name | SEQ ID | NCBI / GenBank / Gen ID | name | SEQ ID | NCBI Reference Sequence / GenBank / Protein ID / | function | |
| *glf* | 17 | M60615.1, base pair position 185-1606 | Glf | 18 | AAA27691.1 | glucose transport protein | *Zymomonas mobilis* |
| *galP* | 20 | 947434 | GalP | 21 | NP_417418.1 | galactose:H(+) symporter | *E. coli* |
| *bglF* | 23 | AF508972.1, base pair position 1320-3176 | BglF | 24 | AAO15901.1 | Ell component of the β-glucoside-PTS | *C. glutamicum R* |

### Example 8

### Construction of plasmids pK18mobsacB_P1-glf_zm, pK18mobsacB_P1-galP_ec and pK18mobsacB_P1-bglF_cgR

Plasmids pK18mobsacB_P1-*glf*_zm, pK18mobsacB_P1-*galP*_ec and pK18mobsacB_P1-*bglF*_cgR were constructed to enable incorporation of the heterologous *glf* gene, *galP* gene and *bglF* gene in lysine producing strain DM2042_*pntAB*_cu. The plasmids are based on the mobilizable vector pK18mobsacB described by Schäfer et al. (Gene 145, 69-73, 1994).

For construction of pK18mobsacB_P1-*glf*_zm, pK18mobsacB_P1-*galP*_ec and pK18mobsacB_P1-*bglF*_cgR, the P1-*glf*_zm polynucleotide according to SEQ ID NO: 19 (carrying intergenic sequences, promotor P1 and *glf* gene derived from *Zymomonas mobilis*), the P1-*galP*_ec polynucleotide according to SEQ ID NO: 22 (carrying intergenic sequences, promotor P1 and *galP* gene derived from *Escherichia coli*) and the P1-*bglF*_*cgR* polynucleotide according to SEQ ID NO: 25 (carrying intergenic sequences, promotor P1 and *bglF* gene derived from *Corynebacterium glutamicum R*) were synthesized and subcloned into pK18mobsacB by GeneArt (ThermoFisher Scientific (Waltham, USA)).

The intergenic sequences of SEQ ID NO: 17, SEQ ID NO: 20 and SEQ ID NO: 23 derived from *Corynebacterium glutamicum* represent homologous sequences which enable to incorporate the heterologous *glf*, *galP* and *bglF* genes by homologous recombination.

To assemble the plasmids pK18mobsacB_P1-*glf*_zm, pK18mobsacB_P1-*galP*_ec and pK18mobsacB_P1-*bglF*_cgR the following steps were done by GeneArt: The two polynucleotides i.e., the vector pK18mobsacB and the corresponding polynucleotide P1-*glf*_zm, P1-*galP*_ec *and* P1-*bglF*_cgR were treated with 2 restriction enzymes (see Table 7 below), ligated and the ligation mixture used to transform *E. coli.*

DNA of plasmids pK18mobsacB_P1-*glf*_zm, pK18mobsacB_P1-*galP*_ec and pK18mobsacB_P1-*bglF*_cgR were isolated from transformants and the polynucleotide P1-*glf*_zm, P1-*galP*_ec and P1-*bglF*_cgR created within pK18mobsacB were analyzed by Sanger sequencing.

**Table 7: Construction of plasmids pK18mobsacB_P1-glf_zm, pK18mobsacB_P1-galP_ec and pK18mobsacB_P1-bglF_cgR**

| polynucleotide | | construction | |
|---|---|---|---|
| name | SEQ ID | restriction enzymes | construct |
| P1- *glf*_zm | 19 | Xmal, Sbfl | pK18mobsacB_P1-*glf*_zm |
| P1-*galP*_ec | 22 | Xmal, Sbfl | pK18mobsacB_P1-*galP*_ec |
| P1-*bglF*_cgR | 25 | Xmal, Sbfl | pK18mobsacB_P1-*bglF*_cgR |

### Example 9

### Construction of strain DM2042_pntAB_cu_P1-glf_zm, DM2042_pntAB_cu_P1-galP_ec and DM2042_pntAB_cu_P1-bglF_cgR

The plasmids pK18mobsacB_P1-*glf*_zm, pK18mobsacB_P1-*galP*_ec and pK18mobsacB_P1-*bglF*_cgR obtained in example 8 were used to incorporate the heterologous gene *glf*_zm derived from *Zymomonas mobilis, galP*_ec derived from *E. coli* and *bglF*_cgR derived from *Corynebacterium glutamicum R,* respectively.

Chemically competent cells of *E. coli* strain S17-1 were transformed with plasmid DNA of pK18mobsacB_P1-*glf*_zm, pK18mobsacB_P1-*galP*_ec and pK18mobsacB_P1-*bglF*_cgR. The presence of the plasmids obtained in example 8 were analyzed by Sanger sequencing, respectively.

For conjugal transfer into the strain DM2042_*pntAB*_cu the modified conjugation method of Schäfer et al. (Journal of Bacteriology 172, 1663 - 1666, 1990) as described in materials and methods was used. Subsequently selection of transconjugant clones was done by identification of saccharose resistance and kanamycin sensitivity phenotype.

Transconjugant clones of DM2042_*pntAB*_cu carrying the heterologous *glf*_zm gene, *galP*_ec gene or *bglF*_cgR gene were selected according to chromosome sequence analysis. The strains carrying the heterologous *glf*_zm gene was called and DM2042_*pntAB*_cu_P1-*glf*_zm.

The strain carrying the heterologous *galP*_ec gene was called DM2042_*pntAB*_cu_P1-*galP*_ec. The strain carrying the heterologous *bglF*_cgR gene was called DM2042_*pntAB*_cu_P1-*bglF*_cgR.

A glycerol stock culture of the transconjugant clones was prepared and used as starting material for further investigations.

### Example 10

### L-lysine production and productivity

Strains DM2042 (reference), DM2042_P1-*ioIT* carrying the genetically modified promotor P1 upstream to *iolT2*, DM2042_*pntAB*_ec carrying the *pntAB*_ec genes derived from *E. coli,* DM2042_*pntAB*_ec_P1-*ioIT* carrying the *pntAB*_ec genes derived from *E. coli and* the genetically modified promotor P1 upstream to *iolT2*, strain DM2042_*pntAB*_cu carrying the *pntAB*_cu genes derived from *C. urealyticum and* strain DM2042_pntAB_cu carrying the *pntAB*_cu genes derived from *C. urealyticum* and the genetically modified promotor P1 upstream to *ioIT2* were analyzed for their ability to produce L-lysine from glucose by batch cultivation using the cultivation system according to Wouter Duetz.

As medium CGXII medium with 7,5 g/l CSL and containing 20 g/l glucose as carbon source was used. The cultures were incubated until complete consumption of glucose as confirmed by glucose analysis using blood glucose-meter. The cultivation time until complete sugar depletion, the concentration of L-lysine and the optical density OD660 were determined. The result of the experiment is presented in Table 8.

**Table 8: L-lysine production and productivity.**

| strain | OD660 | L-lysine¹ (g/l) | Time of sugar depletion (h)² | Productivity (Lys g/l/h) |
|---|---|---|---|---|
| DM2042 | 5,4 | 8,8 | 18,6 | 0,47 |
| DM2042_P1-*iolT* | 8,2 | 6,0 | 12,9 | 0,46 |
| DM2042_*pntAB*_ec | 5,1 | 9,3 | 19,9 | 0,47 |
| DM2042_*pntAB*_ec_P1-*iolT* | 7,2 | 6,9 | 13,4 | 0,51 |
| DM2042_*pntAB*_cu | 4,7 | 9,9 | 21,1 | 0,47 |
| DM2042_*pntAB*_cu_P1-*iolT* | 6,4 | 8,4 | 14,7 | 0,57 |

| | | | | |
|---|---|---|---|---|
| ¹ as L-lysine x HCl ² cultivation time until glucose = 0 g/l | | | | |

Data represent mean values of four independent cultivations.

The experiment shows that expression of heterologous *pntAB_ec* and *pntAB*_cu genes in strains DM2042_*pntAB*_ec and DM2042_*pntAB*_cu result in increased L-lysine titer (g/l) as compared to the parental strains DM2042.

Indeed, the overexpression of *iolT* results in decreased L-lysine titer (g/l) in experiments with strains DM2042_*P1*-*iolT*, DM2042_*pntAB*_ec_*P1-ioIT* and DM2042_*pntAB*_cu_*P1-iolT* in comparison to the respective strains without lolT overexpression but harboring the same pntAB variant.

Surprisingly, the combination of heterologous *pntAB* gene and overexpression of *iolT* results in a strong increase of productivity.

### Example 11

### Comparison of the L-lysine production and productivity using strains with further different sugar uptake systems

Strains DM2042_*pntAB*_cu (reference) carrying the *pntAB*_cu genes derived from *C. urealyticum*, DM2042_*pntAB*_cu_P1-*ioIT* carrying the *pntAB*_cu genes derived from *C. urealyticum* and the genetically modified promotor P1 upstream to *ioIT2*, DM2042_*pntAB*_cu_P1-*glf*_zm carrying the *pntAB*_cu genes derived from *C. urealyticum* and the *glf*_zm gene derived from *Zymomonas mobilis,* DM2042_*pntAB*_cu_P2-*ptsG* carrying the *pntAB*_cu genes derived from *C. urealyticum* and the genetically modified promotor P2 upstream to *ptsG*, DM2042_*pntAB*_cu_P1-*galP*_ec carrying the *pntAB*_cu genes derived from *C. urealyticum* and the *galP*_ec gene derived from *E. coli* and strain DM2042_*pntAB*_cu_P1-*bglF*_cgR carrying the *pntAB*_cu genes derived from *C. urealyticum* and the *bglF*_cgR derived from *Corynebacterium glutamicum* were analyzed for their ability to produce L-lysine from glucose by batch cultivation using the cultivation system according to Wouter Duetz.

CGXII medium with 7,5 g/l CSL and containing 20 g/l glucose as carbon source was used for this experiment. The cultures were incubated until complete consumption of glucose as confirmed by glucose analysis using blood glucose-meter. The cultivation time until complete sugar depletion, the concentration of L-lysine and the optical density OD660 were determined. The result of the experiment is presented in Table 9.

**Table 9: L-lysine production and productivity.**

| strain | OD660 | L-lysine¹ (g/l) | Time of sugar depletion (h)² | Productivity (Lys g/l/h) |
|---|---|---|---|---|
| DM2042_*pntAB*_cu | 4,7 | 9,9 | 21,1 | 0,47 |
| DM2042_*pntAB*_cu_P1*-iolT* | 6,4 | 8,4 | 14,7 | 0,57 |
| DM2042_*pntAB*_cu_P1-*glf*_zm | 4,4 | 10,4 | 22,9 | 0,46 |
| DM2042_*pntAB*_cu_P2-*ptsG* | 6,7 | 6,2 | 28,1 | 0,22 |
| DM2042_*pntAB*_cu_P1-*galP*_ec | 5,0 | 9,6 | 20,1 | 0,48 |
| DM2042_*pntAB*_cu_P1*-bglF*_cgR | 4,8 | 10,1 | 21,4 | 0,47 |

| | | | | |
|---|---|---|---|---|
| ¹ as L-lysine x HCI ² cultivation time until glucose = 0 g/l | | | | |

Data represent mean values of four independent cultivations.

The experiment shows that only the overexpression of *iolT* gene in strain DM2042_*pntAB*_cu_P1-*ioIT* results in a strong increase of productivity as compared to the parental strains DM2042_*pntAB*_cu. An increase in productivity has not been seen by analyzing the other strains comprising overexpression of genes *glf, ptsG, galP* and *bglF*, respectively.

## Claims

1. A *C. glutamicum* strain having the ability to produce L-lysine, comprising a heterologous gene encoding a NAD(P)(+) transhydrogenase subunit alpha, a heterologous gene encoding a NAD(P)(+) transhydrogenase subunit beta and a gene encoding a myo-inositol permease that is overexpressed by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

2. The *C. glutamicum strain* of claim 1, wherein the ability to produce L-lysine is achieved by overexpression of genes coding for enzymes having the function of an aspartate-semialdehyde dehydrogenase, an aspartate aminotransferase, a dihydrodipicolinate synthase, a dihydrodipicolinate reductase, a diaminopimelate decarboxylase, an aspartokinase and of a diaminopimelate dehydrogenase, wherein the overexpression is achieved by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

3. The *C. glutamicum strain* of claim 2, wherein the aspartokinase is feedback-resistant.

4. The *C. glutamicum strain* of claims 2 or 3, further comprising a gene coding for a pyruvate carboxylase that is overexpressed by increasing the copy number of the gene, functionally linking the gene with a strong promoter, enhancing the ribosomal binding site, by codon usage optimization of the start codon or of up to the whole gene, inactivation or deletion of a repressor gene, mutation of the repressor binding region of the promotor or by a combination of two or more of these measures.

5. The *C. glutamicum* strain of any of the preceding claims, wherein the amino acid sequence of the NAD(P)(+) transhydrogenase subunit alpha has an identity of at least 60% to the amino acid sequence according to SEQ ID NO: 7.

6. The *C. glutamicum* strain of any of the preceding claims, wherein the amino acid sequence of the NAD(P)(+) transhydrogenase subunit beta has an identity of at least 70% to the amino acid sequence according to SEQ ID NO: 8.

7. The *C. glutamicum* strain of any of the preceding claims, wherein the amino acid sequence of the myo-inositol permease has an identity of at least 57% to the amino acid sequence according to SEQ ID NO: 12.

8. A method for the fermentative production of an L-lysine comprising the steps of cultivating the *C. glutamicum* strain of any of the preceding claims and accumulating L-lysine in the medium to form an L-lysine containing fermentation broth.

9. The method of claim 8, further comprising manufacturing an L-lysine containing product from said fermentation broth.

10. The method of claim 8, further comprising isolating L-lysine from the L-lysine containing fermentation broth.
